# EUROPEAN PATENT SPECIFICATION

(11) **EP 1 679 672 B1**
(45) Date of publication and mention of the grant of the patent: **14.11.2012**
(21) Application number: 05028748.1
(22) Date of filing: 30.12.2005
(51) Int. Cl.: G08B 25/01, A61B 5/00, A61B 5/02, A61B 5/11, A61B 5/024

(54) **Portable pulse monitoring device and method of its operation**
Tragbare Pulsüberwachungsvorrichtung und Betriebsverfahren
Dispositif portable de surveillance de pulsations et son procédé de fonctionnement

(30) Priority: 10.01.2005 DE 202005000310 U
(43) Date of publication of application: 12.07.2006
(73) Proprietor: King, Rudolf C., Dr. jur., 80335 München (DE)
(72) Inventor: King, Rudolf C., Dr. jur., 80335 München (DE)
(74) Representative: Becker Kurig Straus

(56) References cited:
- WO-A-2004/013824
- DE-A1- 19 639 492

## Description

### Field of the Invention

This invention relates to a portable pulse monitoring device, module or mobile terminal device equipped with such a module for constantly measuring or sensing at least one or several physical parameters of a user, particularly pulse or heart rate, and simultaneously determining the position of said user via a satellite based positioning system and for sending out an emergency signal in case of an emergency condition determined by an excessive heart rate and the like, or more generally in case of deviations of measured physical parameter values when compared to predefined threshold values. The measured parameters and positions may be permanently transmitted to a remote server. The present invention further relates to the method of measuring the pulse and/or further physical parameters and determining the position of a user, transmitting those parameters to a remote server for further processing and transmitting an emergency signal when necessary.

### Background of the invention and prior art

Users with known health problems such as cardiac dysrhythmia, high blood pressure or the like have an increased risk of life-threatening cardiovascular emergencies like a heart attack or stroke. In such an emergency a user might still be able to call for help, but due to following unconsciousness, partial paralysis or amentia he might not be able to fully and correctly indicate his current location. However, in all cardiovascular emergencies, the time period until life-support measures are undertaken by trained individuals is critical since permanent damage to heart, brains and vital organs occurs within only a few minutes after the cardiovascular incidence. It is therefore necessary to get help as fast as possible, thus requiring prompt notification of emergency medical services along with exact positioning of the patient.

For diagnosis of the occurrence of a critical medical condition, medical monitors are widely used. Such monitors are capable of measuring one or more physical parameters of a patient and comparing measured parameter values to reference values, which may be defined by the user or medical staff. When the measured values exceed or fall below certain limits, a signal of any kind may be triggered which can lead medical personnel to take appropriate measures. However, most monitors of this kind require constant presence of medical personnel within the signal range of the monitor to ensure that someone will be able to react on a medical emergency. Alternatively, the user himself needs to call for help, such as an ambulance, when the monitor detects abnormal or critical parameter values. As stated above, the user might not be able to indicate his condition or location to rescue services.

It is therefore desired to provide a device which monitors the medical condition of a user and which is capable of giving an alarm and call for professional help when necessary or more precisely, when physical parameter values measured by the device indicate a deterioration of a user's condition.

Devices for measuring pulse and other physical parameters are widely used for medical monitoring purposes. Cuffs for electronically measuring blood pressure and pulse, as an example, are available for both home and professional use. Those usually comprise a inflatable cuff, a manometer unit and a pulse sensor of any kind, such as a microphone. Other medical monitors include e.g. ECG devices to record electrical currents of heartbeats, or non-invasive blood gas monitors to determine oxygen saturation in the blood. The latter may as well be capable of measuring the heartbeat rate. All these monitors are available as portable devices off-the-shelf.

Similarly, mobile terminals or special emergency transmitters which can be used to call help in case of an emergency are well known. Typically they can be activated by a user by means of a key or button and cause an emergency signal to be sent to a predetermined corresponding receiver. Transmission of the emergency signal may for example be effected via a GSM network as a data signal or a conventional phone call to a number stored in the device, such as the local emergency telephone code or a phone number of a responsible caregiver. However, in general the user needs to activate the device himself and possibly talk to emergency services in order to specify his current condition and location.

Satellite based positioning devices may presently be found in a multitude of instruments, either as single units or combined with further functionalities. The system currently used is GPS (Global Positioning System); another concurring system will be set up by the EADS/Astrium group and is planned to be operative in 2008. While the precision of GPS is already around 3m, the future positioning system will supposedly give significantly better resolution. A satellite based positioning means typically comprises a receiver for receiving satellite signals. To determine a current position, signals of at least three positioning satellites need to be received and analysed. The signals received from orbital satellites each include a time signal given by an atomic clock on board of each satellite as well as the orbital elements of each satellite indicating its position. In practice, a fourth satellite signal is needed if the receiver does not include means for determining its exact time. The basic principles of satellite based positioning are well known in the art.

A combination of measuring means, communication means and positioning means will provide a possibility of monitoring a user's condition and automatically notifying rescue services when an emergency has been detected with the ability to easily locate the user and thus to initiate life support measures as soon as possible.

For similar purposes, WO 00/16288 discloses a protection and surveillance system for persons requiring assistance or protection using GPS based positioning, but without physical monitoring of any kind.

Document DE 198 44 296 is an assembly for patient monitoring consisting of a sensor for physiological parameters and a signal processor, as well as a mobile terminal for communication in a cellular mobile communication network, which is capable of cell-based positioning of the mobile terminal on the basis of the respective base station used.

WO 03/041290 gives an mobile phone with an alarm function, wherein an emergency light is activated and an alarm sound is generated when an alarm button is pushed. A user's heart rate may be measured by an included stethoscope unit, but heart rate monitoring or heart rate dependent alarm functionality are not provided.

WO 2004013824 discloses an alarm system comprising an alarm signalling device comprising a transceiver for transmitting an alarm signal. The system comprises a responder device for receiving the alarm signal, the responder device comprising means for signalling an alarm. The alarm signalling device comprises interface means for receiving user information and activating means for activating the transceiver to transmit the alarm signal in response to the user information. The transceiver device further comprises means for receiving a response signal from the responder device.

Document WO 2004/084720 discloses to a system for actively monitoring a patient including at least one body-worn monitoring device that has at least one sensor capable of measuring at least one physiologic parameter and detecting at least one predetermined event. At least one intermediary device is, linked to the body-worn monitoring device by means of a first wireless network and at least one respondent device is linked to said at least one intermediary device by a second wireless network wherein the respondent device is programmed to perform a specified function automatically when the at least one predetermined event is realized. The monitoring device operates to periodically transmit patient status data to the intermediary device but the system predominantly operates in a quiet state, providing very low power consumption.

In the German patent application DE 196 39 492, an automatic help-activation system is disclosed, comprising a mobile phone and GPS system as well as certain measuring or sensing devices, which determines its position by GPS and activates itself when certain criteria are met.

### Summary of the invention

The object of the present invention is to provide a device, module and mobile terminal for monitoring vital signs as a pulse and for simultaneously determining the position of a user, particularly of a user with risk factors as mentioned above, said device also being capable of transmitting the measured data to a remote emergency center, so that in case of emergency an operator at said emergency center is able to give detailed stored information on location and medical condition of the user if abnormalities are detected and contact with the user cannot be established. Such a device should also be simple in handling for the user and provide additional functions to adapt to certain situations of daily life.

The solution according to the invention provides a device having the features of present claim 1 and the method having the steps of claims 25 and 37, wherein advantageous embodiments are disclosed in the dependent claims. A medical monitoring and alarm device, comprising a measuring means for physical parameters, typically provided in the form of a cuff or strap to apply to extremities or chest of a user, means for communication and data transmission to a remote emergency center, a processing means and a positioning means using satellite based positioning.

To keep handling simple for the user, the monitoring device is automatically activated when the pulse measuring cuff or strap is applied, and it is deactivated in the same way when said cuff or strap is taken off. Any further steps and functions occur automatically and do not require intervention by the user.

At predefined periodic intervals, measurements of physical user parameters are conducted automatically. In a preferred embodiment of the invention, the pulse of a user is measured periodically by using a cuff around arm or leg or a chest strap. Simultaneously the position of the user is determined via satellite based positioning. Threshold values are predefined at the monitoring device or at the server, to which measured parameter values are compared. When exceeding or falling below these threshold values, transmission of a distress signal is triggered.

Three emergency levels may be defined to decide on the appropriate action to take: emergency level 1 indicates a situation in which pulse values are suddenly and/or essentially deviating from those stored as predefined standard or threshold values, but the reason or severity of those deviations is yet unknown; emergency level 2 is entered if a user cannot or does not respond to an emergency level 1 distress signal, i.e. if an attempt to establish contact with the user via a radio communication means is unsuccessful; and emergency level 3 indicates that the user himself has triggered the sending of a distress signal to the emergency center by suitable means present on the monitoring device. Therefore, emergency level 1 does not necessarily provoke the notification of a local rescue center, which would be unnecessary and undesired if the user was not in immediate need for help. When unusual parameter values have been detected, the emergency center is notified by sending a distress signal together with the transmitted physical parameters and positioning data. In response to the distress signal, an operator at the emergency center will attempt to make contact with the user through the transmission means and the included speaker and microphone. Thus the user will be able to give further information about his current condition; if he feels well, he can check, under guidance of the operator of the emergency center, whether a malfunction of the monitoring device caused the distress signal. In that case he can bring his device in for repair or alternatively adjust the fit of the measuring device to ensure accurate results. On the contrary, if the user does not respond to the operator or confirms an emergency situation, the operator may contact local rescue services and/or may through the communication means give instructions to the user till the arrival of rescue services.

### Brief description of the drawings

The present invention will hereinafter be described and explained in detail with reference to the enclosed drawings, where
Fig. 1a shows by example a preferred embodiment and the general component setup of the monitoring device according to the invention, where the device is in the form of a cuff to attach to a user's arm or leg;
Fig. 1b shows an alternative embodiment according to the invention with the monitoring device in the form of a chest strap;
Fig. 2 shows by way of example an implementation of the device of the invention in two separate units;
Fig. 3 illustrates the system of the invention comprising a monitoring device and a remote emergency center; and
Fig. 4 shows in a flow chart the steps of the basic method of the invention.

### Detailed description of the invention

The monitoring device according to the invention comprises means for position determination using a satellite based positioning system, means for measuring one or several physical parameters of a user, such as pulse, blood pressure and the like, means for processing measurement data, positioning data and any further data received by or stored in the monitoring device, and means for transmitting data and voice signals to a remote emergency center. Several extensions and enhancements may be added to the basic embodiment of the invention and will also be described in the following.

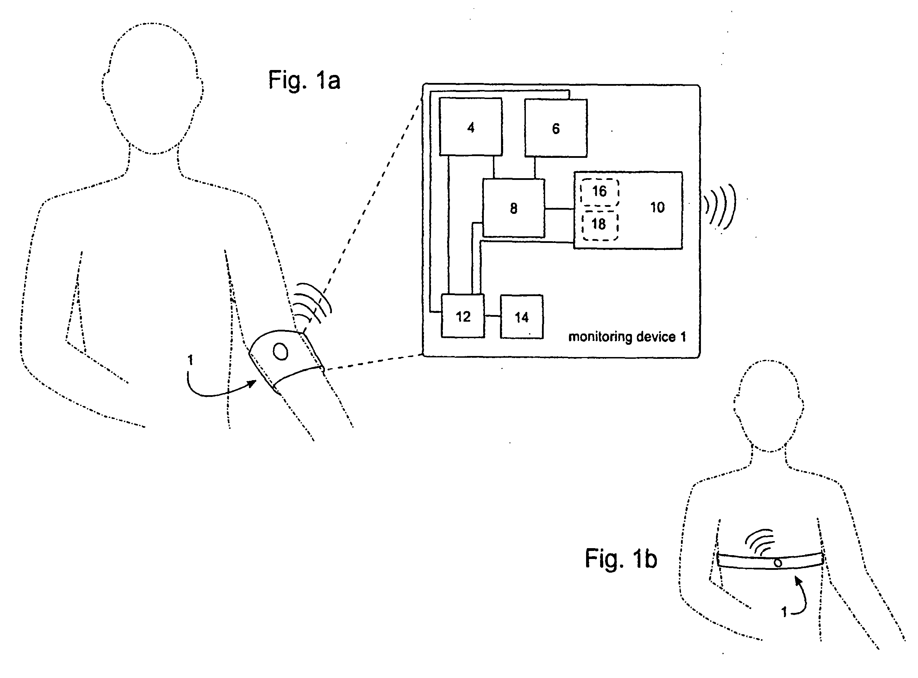

As shown in Fig. 1a, a user may wear the monitoring device 1 as a cuff on his arm, like a conventional blood pressure cuff. However, the device may as well be adapted for wearing on a leg or, in the form of a chest strap, around the chest of a user (shown in Fig. 1b). Included in the measuring cuff is an a on/off-switch 14 that is actuated when fitting the cuff to an extremity of the user and closing it, thereby activating the device; in the same way, the switch is actuated when taking the cuff or strap off and will then deactivate the device. This automatic actuation may be implemented by integrating the switch into the closing mechanism of the cuff. A rechargeable battery 12 is included in the device as a power supply for all components. In the cuff, one or more measuring means 4, 5 are included, which are capable of measuring physical parameters. These may be pulse, blood pressure or other parameters of medical interest.

For positioning purposes, a positioning means 6 is included in the monitoring device. Conventional GPS receivers may be used as positioning means, but in general any suitable satellite based positioning system can be employed in such a device. The positioning means comprises a receiver to receive satellite signals and either processes those signals itself to determine a current position or forwards them to a processing means which will be described below.

The monitoring device further includes a first processing means 8 such as a central processing unit (CPU), which is adapted to convert, process, store and/or forward data supplied by the positioning means and the measuring means 4, 5. This may include, but is not limited to, the parameter values measured by the measuring means, such as the pulse or heart rate and blood pressure, the positioning data from the positioning system, and data received by the communication means or stored in the monitoring device. Since positioning data is typically given in longitude and latitude, conversion into a more comprehensible form is preferred, usually into a conventional address format obtained using a map or database within the processing means. As will be stated below, this format conversion and/or other tasks performed by the processing means 8 may additionally or optionally be taken on by an additional processing means located at the remote emergency center.

Connected to the processing means is a communication means 10 to transmit the processed or forwarded data to a remote emergency center and to establish voice communication between the user and an operator. The communication means may in principle be a mobile phone using a mobile communication network such as a GSM or UMTS network. Generally, any mobile terminal or module may serve as said communication means, as long as it is capable of providing the following functionalities: radio transmission of measuring/positioning data, establishing voice communication to a corresponding terminal of an operator, and establishing voice communication to emergency services. Of course, at least a speaker and a microphone need to be included in the communication means to allow voice communication. While only these basic functions are essential for the monitoring device, the communication means may have further functionalities up to the point of a fully equipped mobile terminal for use in e.g. GSM or UMTS communication systems. As such, the monitoring device may also be integrated as a modification in such a mobile terminal or provided as an add-on module for a standard mobile phone, equipped with a suitable interface to connect to the mobile phone.

With some medical conditions, it may be advisable to not only monitor pulse or heart rhythm but also blood pressure. Thus, in one preferred embodiment, said pulse measuring means 4 may be combined with means for blood pressure measurement. Means of this kind are well known in the art and usually include a cuff that is inflated with air to perform a blood pressure measurement. After both pulse and blood pressure having been determined, again this data is transmitted to a remote server together with positioning data and compared to reference values for pulse and blood pressure, respectively, either at the user device or at the remote server. On activation of the device, preferably a first measurement is initiated immediately. The pulse measurement may be effected by means of the same measuring component as is used for blood pressure measurement, just as in standard available blood pressure meters.

An additional speaker may be comprised in the device to generate a loud alarm signal. The alarm signal may be stored somewhere in the device and may for instance be an alternating sound or some kind of voice calling for help. An alarm signal output may be triggered by the user himself, automatically by the processing means in response to a certain event, or on a command from the emergency center. The alarm signal or voice will attract the attention of people in the adjacency of the user who may be able to help. Optionally, instructions may be given through the speaker by an operator at the emergency center to people on site to e.g. initiate resuscitation. In an alternative embodiment of the invention, the mandatory speaker of the communication device may fulfil those task if the generated sound level is sufficient, such that an additional speaker is not required.

To improve handling and operating of the monitoring device, additional displays, light emitting diodes (LEDs) and similar signalling means may be included in the device. Those may give indication of operating modes, system failures and more; by using LEDs of different colours, such as red, yellow and green LEDs, the operativeness of pulse/blood pressure meter, phone and positioning system may be indicated. A LCD display included in the device may show an operating state of the device, last measured pulse and/or blood pressure values with corresponding measuring time, last position with corresponding time, battery level, pulse signal, signal strength of the mobile communication unit, last data transmission time, and so on.

Situations may arise in which any processes involving sound or noises are undesired, like in a theatre performance or other social events. With this in mind the monitoring device may in a preferred embodiment include an operating mode, further referenced as theatre mode, causing any non-silent processes to stop. To ensure constant and reliable monitoring of the user at that time, responses to certain events may be changed in relation to normal operating mode, which has been described in detail above. Theatre mode may be activated by pressing a muting button included in the device. In detail, switching to theatre mode may cause some or all of the following changes:
- blood pressure measurements are turned off, unless they are feasible in complete silence;
- voice contact in case of emergency level 1 is deactivated, instead a vibration alarm is activated. When abnormal conditions are detected by the monitoring device, the device or part of it starts to vibrate. If the user does not press a specified button, for example said muting button, within a predefined period of time, for instance within 20 seconds, this leads to emergency level 2 and thus to notification of rescue services. If in a preferred embodiment the device additionally comprises a speaker to call for help as set forth above, this speaker does not need to be muted, since the user's need for immediate help should have first priority over any disturbances caused by a loud alarm signal;
- any illuminated parts of the device, such as LEDs or display backlights, are turned off or at least considerably reduced in intensity.

However, the vibration alarm may also be utilized in "normal" operating mode in addition to contact over the phone. In case of two separate units the vibration alarm should preferably be included in the unit that is carried in direct contact with the user's body, which is usually the measuring cuff/strap.

In a further preferred embodiment of the invention, the monitoring device may include a vibration sensor. On detection of a strong, sudden vibration or shock, as it might be caused by activation of an airbag in a car or by bursting glass panes, an accident distress signal similar to the regular distress signal will be transmitted, irrespective of other physical parameter values. This signal will be transmitted immediately, such that the emergency center may be informed of any accident before the device might break down due to severe damage. If then contact with the user cannot be established, a serious emergency can be assumed. The vibration sensor may be separate from the remaining of the device, and the user may attach it e.g. somewhere in a car, like on the dashboard or next to the airbag.

An additional emergency button may be comprised in a preferred embodiment of the invention. This button will be incorporated in the cuff and may be actuated by a strong blow on the cuff performed by the user. This is especially useful when a user himself realizes an deterioration of his condition, such as sudden pain, nausea or dizziness. Actuation of said emergency button will immediately trigger emergency level 3, which will lead to automatic notification of rescue services while specifying the current whereabouts of the user in form of an address obtained by analysing the last transmitted position data. After this notification or simultaneous to it an emergency center operator will attempt to establish contact with the user in order to check whether he is conscious and to eventually gather further information about his condition for passing the same on to rescue services. As before, the distress signal will be transmitted by a radio communication means of the monitoring device. Notification of rescue services may be carried out by the monitoring device itself, or the distress signal is transmitted to the emergency center and may there trigger an automatic emergency call. Any information stored at the server of said emergency center may be suitably converted, such as GPS positioning data to an address, and then forwarded to rescue services.

There is also the option of an embodiment with no pulse monitoring, but only the shock sensitive emergency button as described in the preceding paragraph. This would be sufficient for someone who does not suffer from any cardiovascular disease, but some kind of medical condition which is not detectable by pulse monitoring, such as a condition with suddenly occurring faints.

When travelling on an airplane, use of mobile phones and similar radio communication devices is generally prohibited. Thus an airplane operating mode may be implemented, which may be activated by a button. In airplane mode, the communication means and positioning means is deactivated, while the detection of abnormal parameter values (medical emergency) triggers the output of an alarm signal. Position determination would be possible in an airplane, but does not supply any valuable information, and since the airplane personnel is the only help available and rescue services do not need to be informed, positioning would not make sense in an airplane. As in normal mode, physical parameters such as pulse and/or blood pressure will be monitored and repeatedly compared in the processing means to maximum and minimum threshold values stored in the device. When values beyond these thresholds are detected, the user is notified of this by a vibration alarm as described above or by an audible signal through a speaker included in the device. The audible signal may be some kind of alarm sound or a voice message stored in the device. If the user does not identify the incident as a false alarm by pressing a defined button, loud alarm signals or voice instructions, again stored in a suitable way on the device, may attract the attention of other passengers and inform them about the emergency.

Since all data about users may be automatically gathered and stored at the remote server, those data may be used for elaboration of medical prognoses and risk calculations. The server may include a computer program for data processing, which is capable of evaluating the trend in pulse and/or blood pressure data measured over a certain period of time and may then give suggestions to the user regarding his further actions, such as taking medication for blood dilution or calling on a hospital for further treatment.

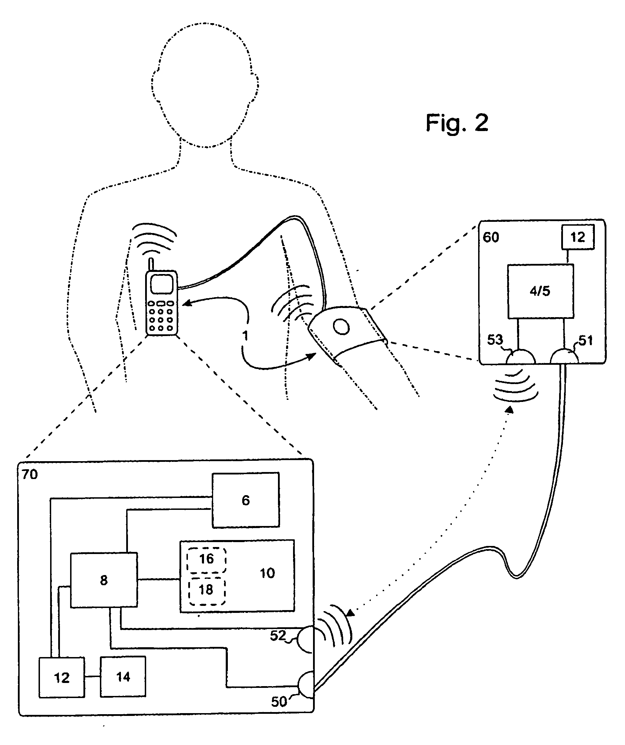

According to a preferred embodiment of the invention, wearing comfort of the device may be enhanced by implementing the measuring means 4, 5 for pulse and/or blood pressure and the remaining components of the monitoring device, such as communication means, processing means and positioning means, in two separate units 60 and 70, as shown in Figure 2. Only the measuring means needs to be placed directly on the user within a first unit 60; any other components may be located, for example, in a second unit 70 in a pocket of the user. The actual measuring unit may in this manner be considerably smaller when some components are enclosed in an extra unit. The two units 60 and 70 need to be interconnected for processing of the data gained from the measurement device; this connection may be wire-based or wireless. To this end, wire-based (50, 51) or wireless interfaces (52, 53) are included in both units.

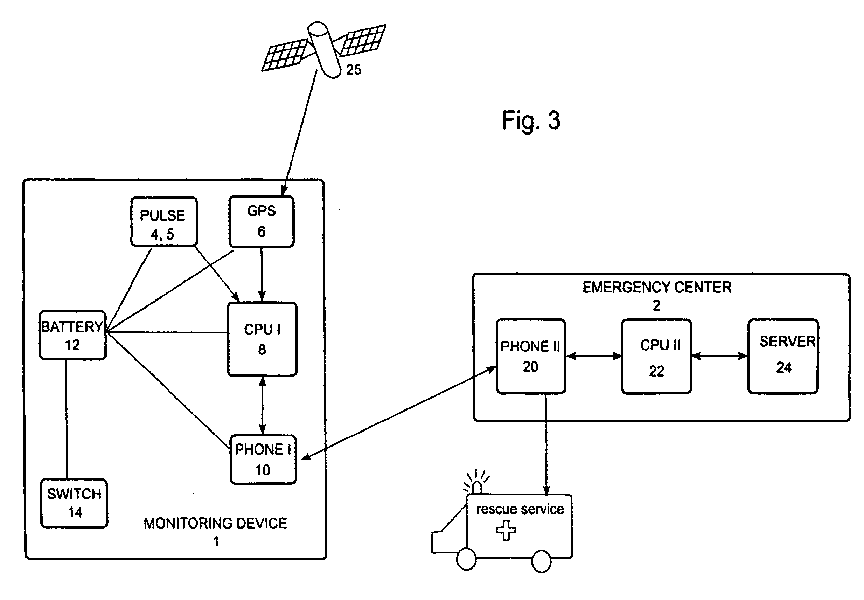

In figure 3, the complete system of the invention can be seen, comprising a monitoring device 1 as described above and a remote emergency center 2. Data may be transmitted between those two via a communication means 10 of the device and a corresponding communication means 20 at the emergency center 2. As stated above, these communication means 10 and 20 may be phones of conventional type, specifically a mobile phone in the monitoring device 1 and any kind of phone at the emergency center 2. The only requirement is that these communication means or phones 10 and 20 are both capable of transmitting and receiving speech and data between each other and to make a call to local rescue services. It should be noted that the emergency center will in general be in charge of a multitude of users or rather their monitoring devices. Each monitoring device 1 will thus repeatedly be transmitting data to the emergency center 2, where it is processed and stored.

The positioning means 6 of the monitoring device 1 uses satellite based positioning, which operates on basis of a set of orbital satellites transmitting signals that are picked up by the positioning means and allow determination of its position by triangulation. Thus, the positioning means 6 searches for positioning satellites 25 when turned on and subsequently receives signals from several satellites. From the received signals it is able to determine the current position, giving longitude and latitude down to a precision of angular seconds.
The processing tasks described for the first processing means 8 of the monitoring device 1 may alternatively be performed at a second processing means 22 (CPU II) located in the emergency center 2. A server 24 is connected to the second processing means 22, where all data received in transmissions may be stored. In addition, databases and programs may be stored at the server 24 which are necessary for operation of the monitoring devices 1. These are, amongst others, threshold values for all users assigned to the emergency center in question, mapping data to gain addresses from the positioning data given as longitude and latitude, programs to evaluate stored user data and analyse medical risk factors and the like.

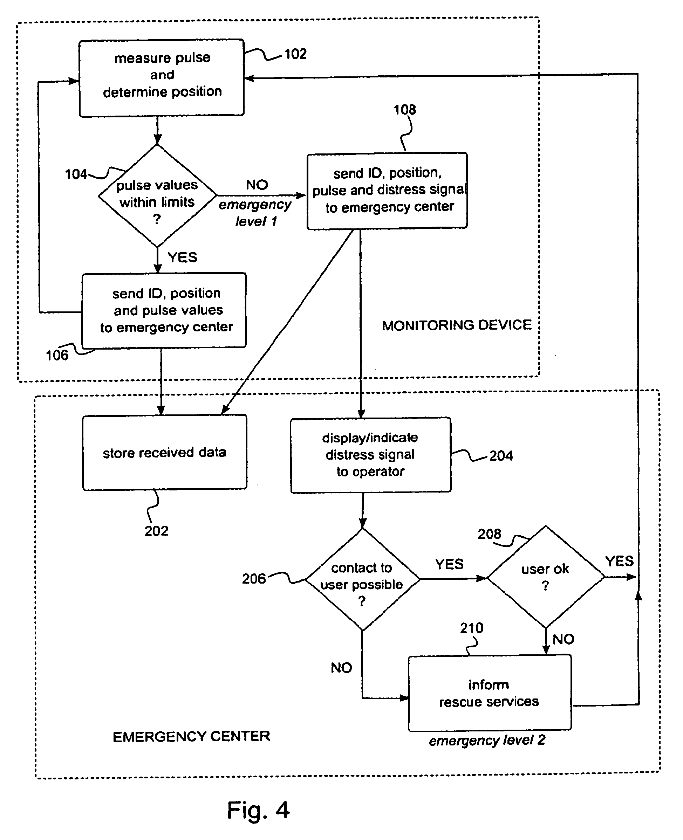

Figure 4 shows in a flow chart the basic method according to the invention by way of example. The process starts at step 102, where an initial determination of a user's pulse and of his current position takes place, using said pulse measuring means (4) and said positioning means as described above. In step 104 then, it is checked by the processor means whether the measured pulse values are within predefined limits or thresholds stored at the monitoring device. If limits are kept, the position data and measured parameter values will be sent to the emergency center (step 106), where they will be received and stored at a server in step 202. If however measured parameter values are below or above said predefined threshold range, this triggers emergency level 1 as described above. A distress signal will be generated by the processing means, and in step 108 this distress signal will be transmitted to the emergency center together with the current position data and the measured pulse values. Transmission of those data to the emergency center is preferably carried out by means of a radio communication link. To identify the user associated with the parameter values, each transmission additionally includes an identifier, which may include a device identification and/or a user identification (ID) to unambiguously associate any values and distress signals with a certain user. When the data has been received at the emergency center, pulse values and position data are again stored at said server, while the distress signal is indicated to an operator of the emergency center in an appropriate way, such as an audible or visible alarm signal (step 204). Thereupon, the operator will attempt to establish contact with the user via any communication device (step 206). If contact with the user cannot be established, it seems probable that he is unconscious and immediately needs help. The operator will first determine the last stored position of the user and then initiate the notification of local rescue services (step 210). He may provide useful information to them, such as the address where the user is located andhis medical data measured by the device and/or stored at the server. If the operator is able to establish contact to the user, he may ask questions about his condition and, if the user feels comfortable (step 208), try to find reasons for the false alarm. A false alarm may be caused by faulty attachment of the measuring cuff, low battery power or similar reasons. However, if the user is able to speak to the operator and confirms an emergency situation, the operator shall again notify rescue services as represented by step 210 and may further give instructions to the user till the arrival of help.
Step 106, which is the transmitting of pulse and position data when threshold values are met, may optionally be left out in one modification of the method, such that transmission of the data to the emergency center is only effected in case of emergency.

Upon activation of the device, the search for satellites is initiated. At the same time, possibly before contact with the positioning satellite has been established, a first pulse measurement is performed and the identifier, including a device identification and/or a user identification, is sent to the emergency center together with the measured pulse values (first transmission without position). After satellite contact has been established, a data signal is send via the mobile communication means to the emergency center, including identification numbers, position and measured pulse values (first transmission with position).
Similarly, on deactivation of the device, a last transmission to the emergency center includes the most recent measured physical parameter value, the most recent determined position, the identifier as described above and a deactivation notification signal, i.e. a signal for notifying the emergency center or rather an operator that the device will now be deactivated by the user.

As will be understood, various modifications of the features described in here can be made. For instance, switching between operating modes such as normal mode, airplane mode and theatre mode may be effected by different buttons or by pressing one single button a various number of times. Also, many of the embodiments described above can be combined with each other and enhanced in various ways. Though only one or two measuring means are mentioned and shown in most of the examples, additional measuring means for other physical parameters may as well be included in the device and may be operating in a similar way to those described. Further, all embodiments of the system and device of the invention may be included in one single unit, in a system of two separate units or in a module utilizable with a mobile terminal. The radio communication systems mentioned for transmitting data between monitoring device and the emergency center are given by way of example only, and it is obvious that any radio communication system which allows long-range contact would be adequate for this application.

## Claims

1. A portable monitoring device (1) for monitoring at least one physical parameter value of a user and transmitting a distress signal on the basis thereof, comprising:
at least one measuring means (4, 5) for measuring said at least physical parameter of a user;
positioning means (6) for satellite based position sensing of said device;
processing means (8) connected to said measuring means (4, 5) and said positioning means (6);
radio communication means (10), connected to said processing means (8), for transmitting data and/or speech;
whereby
said positioning means (6) is configured to periodically determine a current position of said device (1),
said at least one measuring means (4, 5) is configured to automatically sense said physical parameter value of said user,
said processing means (8) is connected to said positioning (6) and said measuring (4, 5) means, and is further configured to read said at least one physical parameter value from said measuring means (4, 5) and to compare said at least one measured parameter value to predetermined threshold values; and wherein said processing means (8) is configured to generate said distress signal when said at least one measured parameter value falls outside said threshold values;
**characterized in that**
said communication means (10) being configured to repeatedly transmit said at least one sensed parameter value together with an identifier, along with current position data obtained by said positioning means (6), and further configured to transmit said distress signal together with said parameter value, current position data and identifier if said distress signal was generated, and
wherein said portable monitoring device further comprises an on/off-switch (14) included in a measuring cuff that is actuated when fitting the cuff to an extremity of the user and closing it, thereby activating the device.

2. The monitoring device (1) according to claim 1, wherein a first measuring means (4) is a heart rate monitor.

3. The monitoring device (1) according to one of the previous claims, wherein a second measuring means (5) is a blood pressure meter.

4. The monitoring device (1) according to any one of the previous claims, wherein said at least one physical parameter value is measured continuously.

5. The monitoring device (1) according to any one of claims 1 to 4, wherein said at least one physical parameter value is measured in periodic intervals.

6. The monitoring device (1) according to any one of the previous claims, wherein said communication means (10) has at least functionality for radio voice communication between said user and a predefined remote center, for voice communication to rescue services and for data signal transmission.

7. The monitoring device according to any one of the previous claims, wherein said communication means (10) is usable with a cellular communication system and capable of determining characteristics of a currently used cell.

8. The monitoring device (1) according to any one of the previous claims, wherein said positioning means (6) comprises a GPS receiver.

9. The monitoring device (1) according to any one of the previous claims, further comprising a vibration sensor sensitive to shocks or strong vibrations of said monitoring device (1).

10. The monitoring device (1) according to claim 9, wherein said processing means (8) is configured to trigger a distress signal in response to a detection of any shock or vibration by said vibration sensor above a predetermined vibration level.

11. The monitoring device (1) according to any one of the previous claims, wherein said monitoring device is in the form of a strap applicable around the chest of a user.

12. The monitoring device (1) according to any one of the previous claims, wherein said device is subdivided into separate first (60) and second (70) units.

13. The monitoring device (1) according to claim 12, wherein said first unit (60) comprises a first wire-based interface (51), and said second unit comprises a corresponding second wire-based interface (50), said first and second wire-based interfaces (50, 51) being connectable by a wire connection for data transmission between said first and second units.

14. The monitoring device according to claim 12 or 13, wherein said first unit (60) comprises a first wireless interface (53), and said second unit comprises a corresponding second wireless interface (52), said first and second wireless interfaces (53, 52) being connectable via a wireless communication connection for data transmission between said first and second units.

15. The monitoring device according to any one of claims 12 to 14, wherein said measuring means is included in said first unit, while said transmission means, positioning means and processing means are included in said second unit.

16. The monitoring device according to any one of claims 12 to 15, wherein said second unit is a module applicable to a standard mobile phone by means of an interface.

17. The monitoring device according to any one of claims 12 to 15, wherein said second unit is an integral part of a standard mobile phone having at least functionality for voice communication between said user and a predefined remote station, voice communication to rescue services and data signal transmission using a mobile communication network.

18. The monitoring device (1) according to any one of the previous claims, further comprising at least one light emitting diode (34) for indicating an operating state of said device.

19. The monitoring device (1) according to any one of the previous claims, further comprising at least one display screen (36) arranged to display one or more of the following information: said at least one measured physical parameter value and measuring time of said physical parameter value, position and time of last positioning, battery level, time of last data transmission.

20. The monitoring device (1) according to any one of the previous claims, further comprising an additional speaker (42) which can be activated by said processing means (8) or by a command received via said communication means (10).

21. The monitoring device (1) according to anyone of the previous claims, further comprising an airplane mode switch, actuation of which deactivates said positioning means and said communication means and activates a speaker or reversely reactivates said positioning means and deactivates said speaker.

22. The monitoring device (1) according to any one of the previous claims, further comprising a muting switch, actuation of which deactivates said blood pressure meter and a speaker (18) of said communication means.

23. The monitoring device (1) according to any one of the previous claims, further comprising a vibration alarm for giving a vibration signal when said at least one measured parameter value falls outside said threshold values.

24. The monitoring device (1) according to claim 23, further comprising a muting switch, actuation of which deactivates said blood pressure meter and a speaker (18) of said communication device and activates said vibration alarm.

25. A method of monitoring at least one physical parameter value of a user and for transmitting a distress signal on the basis thereof using a portable monitoring device (1), comprising the steps of
actuating an on/off-switch (14) included in a measuring cuff when fitting the cuff to an extremity of the user and closing it, thereby activating the device using a portable monitoring device (1);
periodically determining a current position of said user (102) through positioning means (6) for satellite based position sensing included in the device (1);
measuring said at least one physical parameter value of said user (102);
reading said at least one measured parameter value and comparing (104) it to associated lower and/or upper threshold values;
repeatedly transmitting (106) said at least one measured parameter value, an identifier and said position to a remote emergency center by a radio communication means;
and responsive to said at least one parameter value exceeding said upper threshold value or falling below said lower threshold value :
generating a distress signal; and
transmitting (108) said distress signal together with said at least one measured parameter value, said identifier and said position to said remote emergency center.

26. The method according to claim 25, wherein said step of determining a current position is performed by a satellite based positioning means.

27. The method according to claim 25, wherein said step of determining a current position is performed via cell based positioning in a cellular communication network.

28. The method according to any one of claims 25 to 27, wherein said at least one measured parameter value and said current position are converted to a format useable by said communication means before being transmitted by said communication means.

29. The method according to any one of claims 25 to 28, wherein said at least one measured parameter value and said current position are encoded before being transmitted by said communication means.

30. The method according to any one of claims 25 to 29, wherein said at least one measured physical parameter value is the heart rate and/ or blood pressure of a user.

31. The method according to any one of claims 25 to 30, wherein all steps are reiterated until said monitoring device is deactivated.

32. The method according to any one of claims 25 to 31, further comprising, upon activation of the monitoring device, the initial steps of:
- detecting a positioning signal;
- performing an initial measurement of said at least one physical parameter; and
- transmitting said identifier and said at least one measured parameter value to a remote emergency center.

33. The method according to any one of claims 25 to 32, on deactivation of the monitoring device comprising the step of transmitting to said remote emergency center said identifier, said at least one measured parameter value, the current position and a deactivation notification signal.

34. The method according to any one of claims 25 to 33, wherein a distress signal is transmitted to said emergency center responsive to actuation of a button included in said monitoring device.

35. System for monitoring medical parameters of a user, comprising a monitoring device according to any one of the claims 1 to 24, and further comprising a remote emergency center, including a second communication means, a second processing means connected to said second communication means and a server connected to said second processing means; **characterized in that**
said second communication means at said emergency center and said first communication means of said monitoring device are capable of communicating with each other;
said server being capable of storing and outputting data from and to said second processing means, respectively; and
said second processing means is capable of encoding, converting and processing data received from said second communication means or said server.

36. The system according to claim 35, further comprising a component for evaluating and calculating a user's risk for medical emergencies based on any physical parameter data received from said monitoring device and stored at said server.

37. A method of operating a system according to one of claim 35 and 36, comprising the steps of
actuating an on/off-switch (14) included in a measuring cuff when fitting the cuff to an extremity of the user and closing it, thereby activating the device,
periodically determining a current position of said user (102);
automatically measuring said at least one physical parameter value of said user (102);
reading and said at least one measured parameter value and comparing (104) it to associated lower and/or upper threshold values;
repeatedly transmitting (106) said at least one measured parameter value, an identifier and said position to a remote emergency center by a radio communication means;and responsive to said at least one parameter value exceeding said upper threshold value or falling below said lower threshold value :
generating a distress signal;
transmitting (108) said distress signal together with said measured parameter value, said identifier and said position to said remote emergency center;
receiving said at least one measured parameter value, said position, said identifier, and optionally said distress signal at said remote emergency center;
storing said received at least one parameter value and said position at a server at said remote emergency center (202); and
in response to said distress signal, establishing contact (206) with the user via said radio communication means.

38. The method according to claim 37, wherein said received distress signal is audibly and/or visibly indicated (204) to an operator at said remote emergency center.

39. The method according to claim 37 or 38, wherein said step of determining a current position is performed by a satellite based positioning means.

40. The method according to claim 37 or 38, wherein said step of determining a current position is performed via cell based positioning in a cellular communication network.

41. The method according to any one of claims 37 to 40, wherein at least one said physical parameter value and current position determined by said monitoring device are repeatedly transmitted to and stored at said remote server.

42. The method according to any one of claims 37 to 41, wherein said at least one measured parameter value and said current position are converted to a format useable by said communication means before being transmitted by said communication means.

43. The method according to any one of claims 37 to 42, wherein said at least one measured parameter value and said current position are encoded before being transmitted by said communication means.

44. The method according to any one of claims 37 to 43, wherein said at least one measured physical parameter value is the heart rate and/ or blood pressure of a user.

45. The method according to any one of claims 37 to 44, wherein all steps are reiterated until said monitoring device is deactivated.

46. The method according to any one of claims 37 to 45, further comprising, upon activation of the monitoring device, the initial steps of:
- detecting a positioning signal;
- performing an initial measurement of said at least one physical parameter; and
- transmitting said identifier and said at least one measured parameter value to said remote emergency center.

47. The method according to any one of claims 37 to 46, on deactivation of the monitoring device comprising the step of transmitting to said remote emergency center said identifier, said at least one measured parameter value, the current position and a deactivation notification signal.

48. The method according to any one of claims 37 to 47, wherein a speaker on said monitoring device is activated by said monitoring device or said emergency center, emitting a loud alarm signal in response to said distress signal.

49. The method according to any one of claims 37 to 48, wherein a distress signal is transmitted to said emergency center responsive to actuation of a button included in said monitoring device.

50. The method according to any one of claims 37 to 49, further comprising the steps of evaluating measured and stored physical parameter values of a user using stored reference values and profiles, determining risk factors based on these evaluations and sending an instruction message to the user.

## Patentansprüche

1. Tragbare Überwachungsvorrichtung (1) zum Überwachen mindestens eines Körperparameterwerts eines Benutzers und Senden eines Notsignals auf der Grundlage davon, umfassend:
mindestens ein Messmittel (4, 5) zum Messen des mindestens einen Körperparameters eines Benutzers;
ein Positionsbestimmungsmittel (6), um die Position der Vorrichtung satellitengestützt zu erfassen;
ein Verarbeitungsmittel (8), das mit dem Messmittel (4, 5) und dem Positionsbestimmungsmittel (6) verbunden ist;
Funkkommunikationsmittel (10), das mit dem Verarbeitungsmittel (8) verbunden ist, um Daten und/oder Sprache zu übertragen;
wobei
das Positionsbestimmungsmittel (6) konfiguriert ist, periodisch eine gegenwärtige Position der Vorrichtung (1) zu bestimmen,
wobei das mindestens eine Messmittel (4, 5) konfiguriert ist, den Körperparameterwert des Benutzers automatisch zu erfassen,
wobei das Verarbeitungsmittel (8) mit dem Positionsbestimmungsmittel (6) und dem Messmittel (4, 5) verbunden und weiter konfiguriert ist, den mindestens einen Körperparameterwert von dem Messmittel (4, 5) auszulesen und den mindestens einen gemessenen Parameterwert mit vorbestimmten Schwellenwerten zu vergleichen, und
wobei das Verarbeitungsmittel (8) konfiguriert ist, das Notsignal zu erzeugen, wenn der mindestens eine gemessene Parameterwert außerhalb der Schwellenwerte fällt,
**dadurch gekennzeichnet, dass**
das Kommunikationsmittel (10) konfiguriert ist, wiederholt den mindestens einen erfassten Parameterwert zusammen mit einer Kennung nebst den gegenwärtigen Positionsdaten zu senden, die durch das Positionsbestimmungsmittel (6) erhalten wurden, und weiter konfiguriert ist, das Notsignal zusammen mit dem Parameterwert, den gegenwärtigen Positionsdaten und der Kennung zu senden, falls das Notsignal erzeugt wurde, und
wobei die tragbare Überwachungsvorrichtung weiter einen Ein-/Aus-Schalter (14) umfasst, der in einer Messmanschette enthalten ist, der betätigt wird, wenn die Manschette an eine Extremität des Benutzers angelegt und geschlossen wird, wodurch die Vorrichtung aktiviert wird.

2. Überwachungsvorrichtung (1) gemäß Anspruch 1, wobei ein erstes Messmittel (4) ein Herzfrequenzmessgerät ist.

3. Überwachungsvorrichtung (1) gemäß einem der vorstehenden Ansprüche, wobei ein zweites Messmittel (5) ein Blutdruckmesser ist.

4. Überwachungsvorrichtung (1) gemäß einem der vorstehenden Ansprüche, wobei der mindestens eine Körperparameterwert kontinuierlich gemessen wird.

5. Überwachungsvorrichtung (1) gemäß einem der vorstehenden Ansprüche 1-4, wobei der mindestens eine Körperparameterwert in periodischen Intervallen gemessen wird.

6. Überwachungsvorrichtung (1) gemäß einem der vorstehenden Ansprüche, wobei das Kommunikationsmittel (10) mindestens die Funktionalität für Funk-Sprachkommunikation zwischen dem Benutzer und einem vorbestimmten entfernten Zentrum, zur Sprachkommunikation mit Rettungsdiensten und zur Datensignalsendung aufweist.

7. Überwachungsvorrichtung (1) gemäß einem der vorstehenden Ansprüche, wobei das Kommunikationsmittel (10) mit einem zellularen Kommunikationssystem verwendet werden kann und in der Lage ist, Eigenschaften der gegenwärtig verwendeten Zelle zu bestimmen.

8. Überwachungsvorrichtung (1) gemäß einem der vorstehenden Ansprüche, wobei das Positionsbestimmungsmittel (6) einen GPS-Empfänger umfasst.

9. Überwachungsvorrichtung (1) gemäß einem der vorstehenden Ansprüche, weiter umfassend einen Vibrationssensor, der auf Erschütterungen oder starke Vibrationen der Überwachungsvorrichtung (1) anspricht.

10. Überwachungsvorrichtung (1) gemäß Anspruch 9, wobei das Verarbeitungsmittel (8) konfiguriert ist, in Reaktion auf die Erfassung einer Erschütterung oder Vibration durch den Vibrationssensor, die über einem vorbestimmten Vibrationsniveau liegt, ein Notsignal auszulösen.

11. Überwachungsvorrichtung (1) gemäß einem der vorstehenden Ansprüche, wobei die Überwachungsvorrichtung in der Form eines Bandes vorliegt, das um die Brust eines Benutzers angebracht werden kann.

12. Überwachungsvorrichtung (1) gemäß einem der vorstehenden Ansprüche, wobei die Vorrichtung in eine erste Einheit (60) und zweite Einheit (70) unterteilt ist, die voneinander getrennt sind.

13. Überwachungsvorrichtung (1) gemäß Anspruch 12, wobei die erste Einheit (60) eine erste drahtgebundene Schnittstelle (51) und die zweite Einheit (70) eine entsprechende zweite drahtgebundene Schnittstelle (50) umfasst, wobei die erste und die zweite drahtgebundene Schnittstelle zur Datenübertragung zwischen der ersten und der zweiten Einheit durch eine Drahtverbindung verbunden werden können.

14. Überwachungsvorrichtung gemäß Anspruch 12 oder 13, wobei die erste Einheit (60) eine erste drahtlose Schnittstelle (53) und die zweite Einheit eine entsprechende zweite drahtlose Schnittstelle (52) umfasst, wobei die erste und die zweite drahtlose Schnittstelle (53, 52) zur Datenübertragung zwischen der ersten und der zweiten Einheit über eine drahtlose Kommunikationsverbindung verbunden werden können.

15. Überwachungsvorrichtung gemäß einer der Ansprüche 12 bis 14, wobei das Messmittel in der ersten Einheit enthalten ist, während das Sendemittel, das Positionsbestimmungsmittel und das Verarbeitungsmittel in der zweiten Einheit enthalten sind.

16. Überwachungsvorrichtung gemäß einer der Ansprüche 12 bis 15, wobei die zweite Einheit ein Modul ist, das durch eine Schnittstelle an einem Standard-Mobiltelefon angebracht werden kann.

17. Überwachungsvorrichtung gemäß einer der Ansprüche 12 bis 15, wobei die zweite Einheit ein integraler Teil eines Standard-Mobiltelefons ist und mindestens Funktionalitäten für Sprachkommunikation zwischen dem Benutzer und einer vorbestimmten entfernten Station, Sprachkommunikation zu Rettungsdiensten und Datensignalsendungen unter Verwendung eines Mobilkommunikationsnetzwerks aufweist.

18. Überwachungsvorrichtung (1) gemäß einem der vorstehenden Ansprüche, weiter umfassend mindestens eine Leuchtdiode (34), um einen Betriebszustand der Vorrichtung anzuzeigen.

19. Überwachungsvorrichtung (1) gemäß einem der vorstehenden Ansprüche, weiter umfassend mindestens einen Anzeigeschirm (36), der eingerichtet ist, eine oder mehrere der folgenden Informationen anzuzeigen: den mindestens einen gemessenen Körperparameterwert und eine Messzeit des Körperparameterwerts, Position und Zeit der letzten Positionsbestimmung, ein Batterieniveau, und die Zeit der letzten Datensendung.

20. Überwachungsvorrichtung (1) gemäß einem der vorstehenden Ansprüche, weiter umfassend mindestens einen zusätzlichen Lautsprecher (42), der durch das Verarbeitungsmittel (8) oder durch einen Befehl aktiviert werden kann, der über das Kommunikationsmittel (10) empfangen wurde.

21. Überwachungsvorrichtung (1) gemäß einem der vorstehenden Ansprüche, weiter umfassend einen Flugzeugmodus-Schalter, dessen Betätigung die Positionsbestimmungsmittel und die Kommunikationsmittel deaktiviert und einen Lautsprecher aktiviert, oder umgekehrt die Positionsbestimmungsmittel aktiviert und den Lautsprecher deaktiviert.

22. Überwachungsvorrichtung (1) gemäß einem der vorstehenden Ansprüche, weiter umfassend einen Stumm-Schalter, dessen Betätigung den Blutdruckmesser und einen Lautsprecher (18) des Kommunikationsmittels deaktiviert.

23. Überwachungsvorrichtung (1) gemäß einem der vorstehenden Ansprüche, weiter umfassend einen Vibrationsalarm, um ein Vibrationssignal auszugeben, wenn der mindestens eine gemessene Parameterwert aus den Schwellenwerten herausfällt.

24. Überwachungsvorrichtung (1) gemäß Anspruch 23, weiter umfassend einen Stumm-Schalter, dessen Betätigung den Blutdruckmesser und einen Lautsprecher (18) der Kommunikationsvorrichtung deaktiviert und den Vibrationsalarm aktiviert.

25. Verfahren zum Überwachen mindestens eines Körperparameterwertes eines Benutzers und zum Senden eines Notsignals auf Grundlage davon, unter Verwendung einer tragbaren Überwachungsvorrichtung (1), umfassend die Schritte:
Betätigen eines Ein-/Aus-Schalters (14), der in einer Messmanschette enthalten ist, wenn die Manschette an eine Extremität des Benutzers angelegt und geschlossen wird, wodurch die Vorrichtung (1) aktiviert wird;
periodisches Bestimmen einer gegenwärtigen Position des Benutzers (102) durch ein Positionsbestimmungsmittel (6) zur satellitengestützten Positionserfassung, das in der Vorrichtung (1) enthalten ist;
Messen des mindestens einen Körperparameters eines Benutzers (102);
Auslesen und Vergleichen (104) des mindestens einen gemessenen Parameterwerts mit zugeordneten unteren und/oder oberen Schwellenwerten;
wiederholtes Senden (106) des mindestens einen gemessenen Parameterwerts, einer Kennung und der Position an ein entferntes Notfallzentrum durch ein Funkkommunikationsmittel; und
in Reaktion darauf, dass mindestens ein Parameterwert den oberen Schwellenwert überschreitet oder unter den unteren Schwellenwert fällt:
Erzeugen eines Notsignals; und
Senden (108) des Notsignals zusammen mit dem mindestens einen gemessenen Parameterwert, der Kennung und der Position an das entfernte Notfallzentrum.

26. Verfahren gemäß Anspruch 25, wobei der Schritt des Bestimmens einer gegenwärtigen Position von einem satellitengestützten Positionsbestimmungsmittel durchgeführt wird.

27. Verfahren gemäß Anspruch 25, wobei der Schritt des Bestimmens einer gegenwärtigen Position über eine zellengestützte Positionsbestimmung in einem zellularen Kommunikationsnetzwerk durchgeführt wird.

28. Verfahren gemäß einem der Ansprüche 25 bis 27, wobei der mindestens eine gemessene Parameterwert und die gegenwärtige Position in ein Format umgewandelt werden, das von dem Kommunikationsmittel verwendet werden kann, bevor sie durch das Kommunikationsmittel gesendet werden.

29. Verfahren gemäß einem der Ansprüche 25 bis 28, wobei der mindestens eine gemessene Parameterwert und die gegenwärtige Position codiert werden, bevor sie durch das Kommunikationsmittel gesendet werden.

30. Verfahren gemäß einem der Ansprüche 25 bis 29, wobei der mindestens eine gemessene Körperparameterwert die Herzfrequenz und/oder ein Blutdruck eines Benutzers ist.

31. Verfahren gemäß einem der Ansprüche 25 bis 30, wobei alle Schritte wiederholt werden, bis die Überwachungsvorrichtung deaktiviert wird.

32. Verfahren gemäß einem der Ansprüche 25 bis 31, weiter umfassend, auf eine Aktivierung der Überwachungsvorrichtung hin, die anfänglichen Schritte:
- Erfassen eines Positionssignals;
- Durchführen einer anfänglichen Messung des mindestens einen Körperparameters; und
- Senden der Kennung und des mindestens einen gemessenen Parameterwerts an ein entferntes Notfallzentrum.

33. Verfahren gemäß einem der Ansprüche 25 bis 32, weiter umfassend, auf eine Deaktivierung der Überwachungsvorrichtung hin, den Schritt:
Senden der Kennung, des mindestens einen gemessenen Parameterwerts, der gegenwärtigen Position und eines Deaktivierungs-Benachrichtigungssignals an das entfernte Notfallzentrum.

34. Verfahren gemäß einem der Ansprüche 25 bis 33, wobei ein Notsignal in Reaktion auf die Betätigung einer Taste, die in der Überwachungsvorrichtung enthalten ist, an das entfernte Notfallzentrum gesendet wird.

35. System zum Überwachen medizinischer Parameter eines Benutzers, umfassend eine Überwachungsvorrichtung gemäß einem der Ansprüche 1 bis 24, und weiter umfassend ein entferntes Notfallzentrum, enthaltend
ein zweites Kommunikationsmittel;
ein zweites Verarbeitungsmittel, das mit dem zweiten Kommunikationsmittel verbunden ist; und
einen Server, der mit dem zweiten Verarbeitungsmittel verbunden ist;
**dadurch gekennzeichnet, dass**
das zweites Kommunikationsmittel bei dem Notfallzentrum und das erste Kommunikationsmittel der Überwachungsvorrichtung miteinander kommunizieren können;
wobei der Server Daten von dem zweiten/an das zweite Verarbeitungsmittel speichern bzw. ausgeben und Daten an das zweite/von dem zweiten Verarbeitungsmittel ausgeben bzw. ausgeben kann; und
wobei das zweite Verarbeitungsmittel Daten, die von dem zweiten Kommunikationsmittel oder dem Server empfangen wurden, codieren, umwandeln und verarbeiten kann.

36. System gemäß Anspruch 35, weiter umfassend eine Komponente zum Bewerten und Berechnen eines Risikos von medizinischen Notfällen für einen Benutzer, auf Grundlage beliebiger Körperparameterdaten, die von der Überwachungsvorrichtung empfangen wurden und auf dem Server gespeichert sind.

37. Verfahren zum Betreiben eines Systems gemäß einer der Ansprüche 35 bis 36, umfassend die Schritte:
Betätigen eines Ein-/Aus-Schalters (14), der in einer Messmanschette enthalten ist, wenn die Manschette an eine Extremität des Benutzers angelegt und geschlossen wird, wodurch die Vorrichtung aktiviert wird;
periodisches Bestimmen einer gegenwärtigen Position des Benutzers (102);
automatisches Messen des mindestens einen Körperparameterwerts des Benutzers (102);
Auslesen und Vergleichen (104) des mindestens einen gemessenen Parameterwerts mit zugeordneten unteren und/oder oberen Schwellenwerten;
wiederholtes Senden (106) des mindestens einen gemessenen Parameterwerts, einer Kennung und der Position an ein entferntes Notfallzentrum durch ein Funkkommunikationsmittel; und
in Reaktion darauf, dass der mindestens eine Parameterwert den oberen Schwellenwert überschreitet oder unter den unteren Schwellenwert fällt:
Erzeugen eines Notsignals; und
Senden (108) des Notsignals zusammen mit dem gemessenen Parameterwert, der Kennung und der Position an das entfernte Notfallzentrum;
Empfangen des mindestens einen gemessenen Parameterwerts, der Position, der Kennung und optional des Notsignals an dem entfernten Notfallzentrum (202);
Speichern des empfangenen mindestens einen Parameterwerts und der Position an einem Server bei dem entfernten Notfallzentrum (202); und
Herstellen eines Kontakts (206) zu dem Benutzer über das Funkkommunikationsmittel, in Reaktion auf das Notsignal.

38. Verfahren gemäß Anspruch 37, wobei das empfange Notsignal hörbar und/oder sichtbar einem Bediener an dem entfernten Notfallszentrum angegeben wird (204).

39. Verfahren gemäß Anspruch 37 oder 38, wobei der Schritt des Bestimmens einer gegenwärtigen Position von einem satellitengestützten Positionsbestimmungsmittel durchgeführt wird.

40. Verfahren gemäß Anspruch 37 oder 38, wobei der Schritt des Bestimmens einer gegenwärtigen Position über eine zellengestützte Positionsbestimmung in einem zellularen Kommunikationsnetzwerk durchgeführt wird.

41. Verfahren gemäß einem der Ansprüche 37 bis 40, wobei der mindestens eine Körperparameterwert und die gegenwärtige Position, die durch die Überwachungsvorrichtung bestimmt werden, wiederholt an den entfernten Server gesendet werden und auf dem entfernten Server gespeichert werden.

42. Verfahren gemäß einem der Ansprüche 37 bis 41, wobei der mindestens eine gemessene Parameterwert und die gegenwärtige Position in ein Format umgewandelt werden, das von dem Kommunikationsmittel verwendet werden kann, bevor sie durch das Kommunikationsmittel gesendet werden.

43. Verfahren gemäß einem der Ansprüche 37 bis 42, wobei der mindestens eine gemessene Parameterwert und die gegenwärtige Position codiert werden, bevor sie durch das Kommunikationsmittel gesendet werden.

44. Verfahren gemäß einem der Ansprüche 37 bis 43, wobei der mindestens eine gemessene Körperparameterwert die Herzfrequenz und/oder ein Blutdruck eines Benutzers ist.

45. Verfahren gemäß einem der Ansprüche 37 bis 44, wobei alle Schritte wiederholt werden, bis die Überwachungsvorrichtung deaktiviert wird.

46. Verfahren gemäß einem der Ansprüche 37 bis 45, weiter umfassend, auf eine Aktivierung der Überwachungsvorrichtung hin die anfänglichen Schritte auszuführen:
- Erfassen eines Positionssignals;
- Durchführen einer anfänglichen Messung des mindestens einen Körperparameters; und
- Senden der Kennung und des mindestens einen gemessenen Parameterwerts an das entfernte Notfallzentrum.

47. Verfahren gemäß einem der Ansprüche 37 bis 46, weiter umfassend, auf eine Deaktivierung der Überwachungsvorrichtung hin den Schritt auszuführen:
Senden der Kennung, des mindestens einen gemessenen Parameterwerts der gegenwärtigen Position und eines Deaktivierungs-Benachrichtigungssignals an das entfernte Notfallzentrum.

48. Verfahren gemäß einem der Ansprüche 37 bis 47, wobei ein Lautsprecher an der Überwachungsvorrichtung durch die Überwachungsvorrichtung oder das Notfallzentrum aktiviert wird, der in Reaktion auf das Notsignal ein lautes Alarmsignal ausgibt.

49. Verfahren gemäß einem der Ansprüche 37 bis 48, wobei ein Notsignal in Reaktion auf eine Betätigung einer Taste, die in der Überwachungsvorrichtung enthalten ist, an das entfernte Notfallzentrum gesendet wird.

50. Verfahren gemäß einem der Ansprüche 37 bis 49, weiter umfassend die Schritte:
Bewerten gemessener und gespeicherter Körperparameterwerte eines Benutzers unter Verwendung gespeicherter Referenzwerte und Profile,
Bestimmen von Risikofaktoren auf Grundlage dieser Bewertungen, und
Senden einer Anweisungsnachricht an den Benutzer.

## Revendications

1. Dispositif de surveillance portable (1) pour surveiller au moins une valeur de paramètre physique d'un utilisateur et transmettre un signal de détresse en fonction de celle-ci, comprenant :
au moins un moyen de mesure (4, 5) pour mesurer ledit au moins un paramètre physique d'un utilisateur ;
un moyen de positionnement (6) pour la détection de position par voie satellitaire dudit dispositif ;
un moyen de traitement (8) relié au dit moyen de mesure (4, 5) et au dit moyen de positionnement (6) ;
un moyen de radiocommunications (10) relié au dit moyen de traitement (8) pour transmettre des données et/ou des paroles ;
de telle manière que
ledit moyen de positionnement (6) est configuré pour déterminer périodiquement une position actuelle dudit dispositif (1),
ledit au moins un moyen de mesure (4, 5) est configuré pour détecter automatiquement ladite valeur de paramètre physique dudit utilisateur,
ledit moyen de traitement (8) est relié au dit moyen de positionnement (6) et au dit moyen de mesure (4, 5), et est en outre configuré pour lire ladite au moins une valeur de paramètre physique à partir dudit moyen de mesure (4, 5) et comparer ladite au moins une valeur de paramètre mesurée à des valeurs de seuil prédéterminées ; et dans lequel ledit moyen de traitement (8) est configuré pour générer ledit signal de détresse lorsque ladite au moins une valeur de paramètre mesurée passe à l'extérieur desdites valeurs de seuil ;
**caractérisé en ce que**
ledit moyen de communication (10) est configuré pour transmettre à répétition ladite au moins une valeur de paramètre détectée avec un identifiant ainsi que des données de position actuelle obtenues par ledit moyen de positionnement (6), et est en outre configuré pour transmettre ledit signal de détresse avec ladite valeur de paramètre, lesdites données de position actuelle et ledit identifiant si ledit signal de détresse a été généré, et
dans lequel ledit dispositif de surveillance portable comprend en outre un commutateur marche-arrêt (14) inclus dans un manchon de mesure qui est actionné en plaçant le manchon à une extrémité de l'utilisateur et en le fermant, ce qui active le dispositif.

2. Dispositif de surveillance (1) selon la revendication 1, dans lequel un premier moyen de mesure (4) est un moniteur de rythme cardiaque.

3. Dispositif de surveillance (1) selon l'une des revendications précédentes, dans lequel un deuxième moyen de mesure (5) est un dispositif de mesure de pression artérielle.

4. Dispositif de surveillance (1) selon l'une quelconque des revendications précédentes, dans lequel ladite au moins une valeur de paramètre physique est continuellement mesurée.

5. Dispositif de surveillance (1) selon l'une quelconque des revendications 1 à 4, dans lequel ladite au moins une valeur de paramètre physique est mesurée à intervalles périodiques.

6. Dispositif de surveillance (1) selon l'une quelconque des revendications précédentes, dans lequel ledit moyen de communication (10) a au moins une fonctionnalité de communication vocale radio entre ledit utilisateur et un centre distant prédéterminé, pour une communication vocale avec des services de secours et pour une transmission de signal de données.

7. Dispositif de surveillance (1) selon l'une quelconque des revendications précédentes, dans lequel ledit moyen de communication (10) est utilisable avec un système de communication cellulaire et est capable de déterminer des caractéristiques d'une cellule en cours d'utilisation.

8. Dispositif de surveillance (1) selon l'une quelconque des revendications précédentes, dans lequel ledit moyen de positionnement (6) comprend un récepteur GPS.

9. Dispositif de surveillance (1) selon l'une quelconque des revendications précédentes, comprenant en outre un capteur de vibrations sensible aux chocs ou aux fortes vibrations dudit dispositif de surveillance (1).

10. Dispositif de surveillance (1) selon la revendication 9, dans lequel ledit moyen de traitement (8) est configuré pour déclencher un signal de détresse en réponse à une détection d'un choc ou d'une vibration par ledit capteur de vibrations au-dessus d'un niveau de vibrations prédéterminé.

11. Dispositif de surveillance (1) selon l'une quelconque des revendications précédentes, dans lequel ledit dispositif de surveillance se présente sous la forme d'une sangle applicable autour de la poitrine d'un utilisateur.

12. Dispositif de surveillance (1) selon l'une quelconque des revendications précédentes, dans lequel ledit dispositif est subdivisé en des première (60) et deuxième (70) unités distinctes.

13. Dispositif de surveillance (1) selon la revendication 12, dans lequel ladite première unité (60) comprend une première interface câblée (51) et ladite deuxième unité comprend une deuxième interface câblée (50) correspondante, lesdites première et deuxième interfaces câblées (50, 51) pouvant être reliées par une liaison câblée pour une transmission de données entre lesdites première et deuxième unités.

14. Dispositif de surveillance selon la revendication 12 ou 13, dans lequel ladite première unité (60) comprend une première interface sans fil (53), et ladite deuxième unité comprend une deuxième interface sans fil (52) correspondante, lesdites première et deuxième interfaces sans fil (53, 52) pouvant être reliées par l'intermédiaire d'une liaison de communication sans fil pour une transmission de données entre lesdites première et deuxième unités.

15. Dispositif de surveillance selon l'une quelconque des revendications 12 à 14, dans lequel ledit moyen de mesure est inclus dans ladite première unité, alors que ledit moyen de transmission, ledit moyen de positionnement et ledit moyen de traitement sont inclus dans ladite deuxième unité.

16. Dispositif de surveillance selon l'une quelconque des revendications 12 à 15, dans lequel ladite deuxième unité est un module applicable à un téléphone mobile standard au moyen d'une interface.

17. Dispositif de surveillance selon l'une quelconque des revendications 12 à 15, dans lequel ladite deuxième unité fait partie intégrante d'un téléphone mobile standard ayant au moins une fonctionnalité de communication vocale entre ledit utilisateur et une station distante prédéfinie, une fonctionnalité de communication vocale avec des services de secours et une fonctionnalité de transmission de signal de données en utilisant un réseau de communication mobile.

18. Dispositif de surveillance (1) selon l'une quelconque des revendications précédentes, comprenant en outre au moins une diode électroluminescente (34) pour indiquer un état de fonctionnement dudit dispositif.

19. Dispositif de surveillance (1) selon l'une quelconque des revendications précédentes, comprenant en outre au moins un écran d'affichage (36) agencé pour afficher une ou plusieurs des informations suivantes : ladite au moins une valeur de paramètre physique mesurée et l'heure de mesure de ladite valeur de paramètre physique, la position et l'heure du dernier positionnement, le niveau de batterie, l'heure de la dernière transmission de données.

20. Dispositif de surveillance (1) selon l'une quelconque des revendications précédentes, comprenant en outre un haut-parleur supplémentaire (42) pouvant être activé par ledit moyen de traitement (8) ou par une commande reçue par l'intermédiaire dudit moyen de communication (10).

21. Dispositif de surveillance (1) selon l'une quelconque des revendications précédentes, comprenant en outre un commutateur de mode avion dont l'actionnement désactive ledit moyen de positionnement et ledit moyen de communication et active un haut-parleur ou réactive inversement ledit moyen de positionnement et désactive ledit haut-parleur.

22. Dispositif de surveillance (1) selon l'une quelconque des revendications précédentes, comprenant en outre un commutateur de silencieux dont l'actionnement désactive ledit dispositif de mesure de pression artérielle et un haut-parleur (18) dudit moyen de communication.

23. Dispositif de surveillance (1) selon l'une quelconque des revendications précédentes, comprenant en outre une alarme vibratoire pour donner un signal vibratoire lorsque ladite au moins une valeur de paramètre mesurée passe à l'extérieur desdites valeurs de seuil.

24. Dispositif de surveillance (1) selon la revendication 23, comprenant en outre un commutateur de silencieux dont l'actionnement désactive ledit dispositif de mesure de pression artérielle et un haut-parleur (18) dudit dispositif de communication et active ladite alarme vibratoire.

25. Procédé de surveillance d'au moins une valeur de paramètre physique d'un utilisateur et de transmission d'un signal de détresse en fonction de celle-ci, en utilisant un dispositif de surveillance portable (1), comprenant les étapes de :
l'actionnement d'un commutateur marche-arrêt (14) inclus dans un manchon de mesure en plaçant le manchon à une extrémité de l'utilisateur et en le fermant, en activant de ce fait le dispositif (1),
la détermination périodique d'une position actuelle dudit utilisateur (102) par le biais d'un moyen de positionnement (6) pour la détection de position par voie satellitaire inclus dans le dispositif (1) ;
la mesure de ladite au moins une valeur de paramètre physique dudit utilisateur (102) ;
la lecture de ladite au moins une valeur de paramètre mesurée et la comparaison (104) de celle-ci à des valeurs de seuil inférieur et/ou de seuil supérieur associées ;
la transmission à répétition (106) de ladite au moins une valeur de paramètre mesurée, d'un identifiant et de ladite position à un centre d'urgence distant par un moyen de radiocommunication ;
et en réponse au fait que ladite au moins une valeur de paramètre dépasse ladite valeur de seuil supérieur ou descend au-dessous de ladite valeur de seuil inférieur :
la génération d'un signal de détresse ; et
la transmission (108) dudit signal de détresse avec ladite au moins une valeur de paramètre mesurée, ledit identifiant et ladite position au dit centre d'urgence distant.

26. Procédé selon la revendication 25, dans lequel ladite étape de détermination d'une position actuelle est effectuée par un moyen de positionnement par voie satellitaire.

27. Procédé selon la revendication 25, dans lequel ladite étape de détermination d'une position actuelle est effectuée au moyen d'un positionnement par voie cellulaire dans un réseau de communication cellulaire.

28. Procédé selon l'une quelconque des revendications 25 à 27, dans lequel ladite au moins une valeur de paramètre mesurée et ladite position actuelle sont converties dans un format utilisable par ledit moyen de communication avant leur transmission par ledit moyen de communication.

29. Procédé selon l'une quelconque des revendications 25 à 28, dans lequel ladite au moins une valeur de paramètre mesurée et ladite position actuelle sont codées avant leur transmission par ledit moyen de communication.

30. Procédé selon l'une quelconque des revendications 25 à 29, dans lequel ladite au moins une valeur de paramètre physique mesurée est le rythme cardiaque et/ou la pression artérielle d'un utilisateur.

31. Procédé selon l'une quelconque des revendications 25 à 30, dans lequel toutes les étapes sont réitérées jusqu'à la désactivation dudit dispositif de surveillance.

32. Procédé selon l'une quelconque des revendications 25 à 31, comprenant en outre, à l'activation du dispositif de surveillance, les étapes initiales de :
- la détection d'un signal de positionnement ;
- la prise d'une mesure initiale dudit au moins un paramètre physique ; et
- la transmission dudit identifiant et de ladite au moins une valeur de paramètre mesurée à un centre d'urgence distant.

33. Procédé selon l'une quelconque des revendications 25 à 32, comprenant en outre, à la désactivation du dispositif de surveillance, l'étape de la transmission au dit centre d'urgence distant dudit identifiant, de ladite au moins une valeur de paramètre mesurée, de la position actuelle et d'un signal de notification de désactivation.

34. Procédé selon l'une quelconque des revendications 25 à 33, dans lequel un signal de détresse est transmis au dit centre d'urgence en réponse à l'actionnement d'un bouton inclus dans ledit dispositif de surveillance.

35. Système de surveillance de paramètres médicaux d'un utilisateur, comprenant un dispositif de surveillance selon l'une quelconque des revendications 1 à 24, et comprenant en outre un centre d'urgence distant, comprenant un deuxième moyen de communication, un deuxième moyen de traitement relié au dit deuxième moyen de communication et un serveur relié au dit deuxième moyen de traitement ; **caractérisé en ce que**
ledit deuxième moyen de communication au dit centre d'urgence et ledit premier moyen de communication dudit dispositif de surveillance sont capables de communiquer entre eux ;
ledit serveur est capable de stocker et de délivrer des données respectivement en provenance et à destination dudit deuxième moyen de traitement ; et
ledit deuxième moyen de traitement est capable de coder, de convertir et de traiter des données reçues dudit deuxième moyen de communication ou dudit serveur.

36. Système selon la revendication 35, comprenant en outre un composant pour évaluer et calculer un risque d'urgence médicale d'un utilisateur sur la base de données de paramètre physique reçues dudit dispositif de surveillance et stockées dans ledit serveur.

37. Procédé d'exploitation d'un système selon l'une des revendications 35 à 36, comprenant les étapes de :
l'actionnement d'un commutateur marche-arrêt (14) inclus dans un manchon de mesure en plaçant le manchon à une extrémité de l'utilisateur et en le fermant, en activant de ce fait le dispositif,
la détermination périodique d'une position actuelle dudit utilisateur (102) ;
la mesure automatique de ladite au moins une valeur de paramètre physique dudit utilisateur (102) ;
la lecture de ladite au moins une valeur de paramètre mesurée et la comparaison (104) de celle-ci à des valeurs de seuil inférieur et/ou supérieur associées ;
la transmission à répétition (106) de ladite au moins une valeur de paramètre mesurée, d'un identifiant et de ladite position à un centre d'urgence distant par un moyen de radiocommunication ; et
en réponse au fait que ladite au moins une valeur de paramètre dépasse ladite valeur de seuil supérieur ou descend au-dessous de ladite valeur de seuil inférieur :
la génération d'un signal de détresse ; et
la transmission (108) dudit signal de détresse avec ladite au moins une valeur de paramètre mesurée, ledit identifiant et ladite position au dit centre d'urgence distant ;
la réception de ladite au moins une valeur de paramètre mesurée, de ladite position, dudit identifiant, et facultativement dudit signal de détresse au dit centre d'urgence distant ;
le stockage de ladite au moins une valeur de paramètre reçue et de ladite position dans un serveur au dit centre d'urgence distant (202) ; et
en réponse au dit signal de détresse, l'établissement d'un contact (206) avec l'utilisateur par le biais dudit moyen de radiocommunication.

38. Procédé selon la revendication 35, dans lequel ledit signal de détresse reçu est indiqué de manière audible et/ou visible (204) à un opérateur dudit centre d'urgence distant.

39. Procédé selon la revendication 37 ou 38, dans lequel ladite étape de détermination d'une position actuelle est effectuée par un moyen de positionnement par voie satellitaire.

40. Procédé selon la revendication 37 ou 38, dans lequel ladite étape de détermination d'une position actuelle est effectuée au moyen d'un positionnement par voie cellulaire dans un réseau de communication cellulaire.

41. Procédé selon l'une quelconque des revendications 37 à 40, dans lequel ladite au moins une valeur de paramètre physique et ladite position actuelle déterminées par ledit dispositif de surveillance sont transmises à répétition au dit serveur distant où elles sont stockées.

42. Procédé selon l'une quelconque des revendications 37 à 41, dans lequel ladite au moins une valeur de paramètre mesuré et ladite position actuelle sont converties dans un format utilisable par ledit moyen de communication avant leur transmission par ledit moyen de communication.

43. Procédé selon l'une quelconque des revendications 37 à 42, dans lequel ladite au moins une valeur de paramètre mesurée et ladite position actuelle sont codées avant leur transmission par ledit moyen de communication.

44. Procédé selon l'une quelconque des revendications 37 à 43, dans lequel ladite au moins une valeur de paramètre physique mesurée est le rythme cardiaque et/ou la pression artérielle d'un utilisateur.

45. Procédé selon l'une quelconque des revendications 37 à 44, dans lequel toutes les étapes sont réitérées jusqu'à la désactivation dudit dispositif de surveillance.

46. Procédé selon l'une quelconque des revendications 37 à 45, comprenant en outre, à l'activation du dispositif de surveillance, les étapes initiales de :
- la détection d'un signal de positionnement ;
- la prise d'une mesure initiale dudit au moins un paramètre physique ; et
- la transmission dudit identifiant et de ladite au moins une valeur de paramètre mesurée au dit centre d'urgence distant.

47. Procédé selon l'une quelconque des revendications 37 à 46, comprenant en outre, à la désactivation du dispositif de surveillance, l'étape de la transmission au dit centre d'urgence distant dudit identifiant, de ladite au moins une valeur de paramètre mesurée, de la position actuelle et d'un signal de notification de désactivation.

48. Procédé selon l'une quelconque des revendications 37 à 47, dans lequel un haut-parleur sur ledit dispositif de surveillance est activé par ledit dispositif de surveillance ou ledit centre d'urgence, en émettant un signal d'alarme fort en réponse au dit signal de détresse.

49. Procédé selon l'une quelconque des revendications 37 à 48, dans lequel un signal de détresse est transmis au dit centre d'urgence en réponse à l'actionnement d'un bouton inclus dans ledit dispositif de surveillance.

50. Procédé selon l'une quelconque des revendications 37 à 49, comprenant en outre les étapes de l'évaluation des valeurs de paramètre physique mesurées et stockées d'un utilisateur en utilisant des valeurs de référence et des profils stockés, la détermination de facteurs de risque sur la base de ces évaluations et l'envoi d'un message d'instruction à l'utilisateur.
